# EUROPÄISCHE PATENTSCHRIFT

(11) **EP 0 681 567 B1**
(45) Veröffentlichungstag und Bekanntmachung des Hinweises auf die Patenterteilung: **22.04.1998**
(21) Anmeldenummer: 94906162.6
(22) Anmeldetag: 28.01.1994
(51) Int. Cl.: C07C 409/34, C08F 218/16

(54) **DIACYLPEROXIDESTER**
DIACYL PEROXIDE ESTERS
ESTERS DE DIACYLPEROXYDE

(30) Priorität: 29.01.1993 DE 4302523
(43) Veröffentlichungstag der Anmeldung: 15.11.1995
(73) Patentinhaber: Peroxid-Chemie GmbH, 82049 Pullach (DE); WACKER-CHEMIE GMBH, D-81737 München (DE)
(72) Erfinder: DORN, Maximilian, D-82031 Grünwald (DE); HÄGEL, Eberhard, D-82057 Icking (DE); KOHLHAMMER, Klaus, D-84533 Marktl (DE)
(74) Vertreter: Weickmann, Heinrich, Dipl.-Ing.
(86) Internationale Anmeldenummer: EP9400237
(87) Internationale Veröffentlichungsnummer: WO9417037

(56) Entgegenhaltungen:
- DE-A- 2 162 534
- DE-C- 170 727
- FR-A- 1 293 219
- FR-A- 1 538 002

## Beschreibung

Die Erfindung betrifft neue Diacylperoxidester, deren Herstellung und Verwendung.

Die Diacylperoxide der allgemeinen Formel

R-CO-O-O-CO-R

stellen viel verwendete Polymerisationskatalysatoren dar. Die katalytischen Eigenschaften verdanken sie der leichten Bildung von polymerisationsauslösend wirkenden Radikalen. Sie werden normalerweise den zu polymerisierenden Monomeren zugemischt und entfalten ihre katalytische Aktivität unter den angewendeten Polymerisationsbedingungen. So beschreibt die DE-A 21 62 534 Diacylperoxid-Initiatoren, deren Zerfallsprodukte in das Polymer eingebaut werden und ihnen gegen UV-Strahlen stabilisierende Wirkung verleihen. Sie enthalten nur eine polymerisationsfähige Gruppierung im Molekül. Da Polymerisationen vielfach auch in mehreren Stufen durchgeführt werden unter Bildung von Zwischenprodukten, die weiteren Polymerisationsreaktionen ausgesetzt werden, besteht ein Bedarf an Polymerisationskatalysatoren, welche selbst polymerisationsfähig sind und in der Lage sind, eine Copolymerisation mit anderen Monomeren unter Bedingungen, welche noch keinen radikalischen Zerfall ihrer peroxidischen Strukturen hervorrufen, einzugehen, in einpolymerisierter Form aber unter anderen Bedingungen Radikale bilden und so eine Weiterpolymerisation erleichtern.

Von besonderem Interesse sind hierbei zur Radikalbildung geeignete Verbindungen, die mehr als eine polymerisationsfähige Gruppierung im Molekül enthalten. Aufgabe der Erfindung ist es, solche zur Radikalbildung befähigte, mehr als eine olefinische Doppelbindung enthaltende Verbindungen zur Verfügung zu stellen.

Gelöst wird diese Aufgabe erfindungsgemäß durch Diacylperoxidvinylester der allgemeinen Formel I 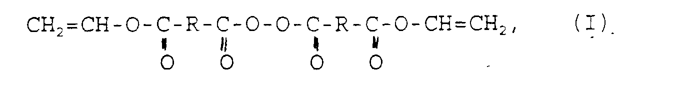 in der R einen Alkylenrest, der geradkettig, verzweigt oder cyclisch sein kann und 2 bis 10 C-Atome enthalten kann oder einen Arylenrest bedeutet.

Die erfindungsgemäßen Verbindungen sind Initiatoren, die in das Polymer einpolymerisieren ohne dabei zu zerfallen (Copolymerisation mit dem Monomeren). Erst bei geänderten Bedingungen, z.B. bei höherer Temperatur, sind die Diacylperoxidgruppen Polymer zur Bildung von Radikalen befähigt, denen sich dann weitere Monomere anlagern können, so daß eine Weiterpolymerisation stattfindet.

Die erfindungsgemäßen Verbindungen weisen eine gute Stabilität auf mit Halbwertzeiten von etwa 10 Stunden bei Temperaturen zwischen etwa 45 und 54°C und können daher als Comonomere im Rahmen von Copolymerisationen bei Temperaturen bis zu etwa 50°C eingesetzt werden ohne daß der Aktiv-Sauerstoffgehalt, der durch die erfindungsgemäßen Verbindungen in das Copolymerisat eingeführt wird, hierdurch sich wesentlich verringert. Bei höheren Temperaturen, insbesondere oberhalb etwa 70°C, entfalten die erfindungsgemäßen Verbindungen InitiatorAktivität und eignen sich besonders gut bei Temperaturen von 80°C und darüber als Polymerisationsinitiatoren. Als Stabilisatorzusätze eignen sich besonders phenolische Verbindungen wie z.B. tert-Butylbrenzkatechin.

Die Herstellung der erfindungsgemäßen Verbindungen erfolgt erfindungsgemäß ausgehend von den entsprechenden Dicarbonsäurehalbestern. Gemäß einer ersten Ausführungsform des erfindungsgemäßen Verfahrens zur Herstellung der Diacylperoxidvinylester der allgemeinen Formel I setzt man eine Monovinyldicarbonsäure der Formel II 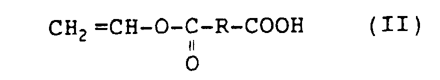 worin R die zu Formel I angegebene Bedeutung hat mit mindestens äquimolaren Mengen an Dicyclohexylcarbodiimid und H₂ O₂ und katalytischen Mengen eines aromatischen Amins unter Kühlung in einem apolaren aprotischen Lösungsmittel um.

Als apolares aprotisches Lösungsmittel wird Toluol bevorzugt, in Betracht kommen aber auch andere aliphatische und aromatische sowie halogenierte Kohlenwasserstoffe. Als katalytisch wirksames aromatisches Amin wird Dimethylaminopyridin bevorzugt. Unter den Verbindungen der Formel II werden diejenigen bevorzugt, in denen R einen Alkylenrest mit 4 oder 6 C-Atomen, einen Cyclohexylenrest oder einen gegebenenfalls substituierten Phenylenrest darstellt. Substituenten am Phenylenrest können geradkettige oder verzweigte Alkylreste mit 1 bis 4 C-Atomen sein. Besonders bevorzugt wird Monovinyladipinsäure als Ausgangsmaterial.

Die Herstellung von Verbindungen der allgemeinen Formel II kann nach dem Verfahren der DE-AS 12 24 93 erfolgen, entweder durch Umesterung mit dem Vinylester einer niederen aliphatischen Carbonsäure oder durch Umsetzung mit Acetylen in Gegenwart eines Katalysators.

Gemäß einer weiteren Ausführungsform des erfindungsgemäßen Verfahrens zur Herstellung der Verbindungen der allgemeinen Formel I setzt man Monovinyldicarbonsäurechlorid der Formel III 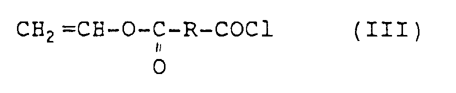 worin R die zu Formel I angegebene Bedeutung hat, mit einer alkalischen wäßrigen H₂ O₂-Lösung bei einer Temperatur von -10 bis +10°C um. Bei dieser Ausführungsform des Verfahrens der Erfindung wird das H₂ O₂ vorzugsweise in einem Überschuß, bezogen auf das Säurechlorid, eingesetzt. Besonders zweckmäßig erwies sich ein molarer Unterschied von 20 bis 40 %.

In bezug auf die bevorzugt eingesetzten Verbindungen der Formel III gilt das zur ersten Ausführungsform des erfindungsgemäßen Verfahrens Angegebene in gleicher Weise. Besonders bevorzugt werden die Verbindungen in denen R = Alkylen mit 4 oder 6 Kohlenstoffatomen, Cyclohexylen oder Phenylen bedeutet. Die Herstellung der Ausgangsverbindungen der allgemeinen Formel III kann erfolgen durch Umsetzung des Monovinylesters der Dicarbonsäure mit Phosphortrichlorid.

Die erfindungsgemäßen Diacylperoxide der allgemeinen Formel I eignen sich insbesondere zur Copolymerisation mit anderen olefinisch ungesättigten Monomeren, insbesondere mit solchen aus der Gruppe Vinylacetat, Ethylen, Styrol, Vinylchlorid, Vinylidenchlorid, Methacrylat und Methylacrylat. Die Copolymerisation wird hierbei zweckmäßig bei Temperaturen von höchstens 50 °C, vorzugsweise 30 bis 45°C durchgeführt um den Aktivsauerstoffgehalt des monomeren Reaktionsteilnehmers der Formel möglichst unverändert zu erhalten. Man erhält so Copolymere mit Aktiv-Sauerstoffgehalt, die sich bei höheren Temperaturen für die weitere Umsetzung unter Entfaltung der Initiatorwirksamkeit der erfindungsgemäßen Komponente der Formel I eignen. Der Anteil an Verbindung der Formel I als Comonomer hängt ab von der gewünschten Initiatoraktivität des so erhaltenen Zwischenproduktes. Im allgemeinen werden 0,5 bis 5 % am Comonomer der allgemein Formel I für die Copolymerisation eingesetzt. Für besondere Zwecke kommen aber auch höhere und niedrigere Zusätze in Betracht.

Das bei dieser Copolymerisation erhaltene Zwischenprodukt zeichnet sich vor allem durch eine geringere Empfindlichkeit und damit höhere Stabilität aus als das Monomer der allgemeinen Formel I selbst und eignet sich daher insbesondere für die Copolymerisation mit anderen Monomeren, insbesondere von Styrol, mit dem Basiscopolymer.

Die folgenden Beispiele erläutern die Erfindung weiter.

### Beispiel 1

### Bis-(vinyladipoyl)-peroxid aus der Säure

Zu einer Lösung von 0,8 Mol (170 g) Dicyclohexylcarbodiimid und 0,04 Mol (5 g) 4-Dimethylaminopyridin in 200 g Toluol gibt man unter Rühren und Kühlen 0,6 Mol (24 g) 85 % H₂ O₂. Bei +5 bis 10°C tropft man eine Lösung von 0,5 Mol (87 g) Monovinyladipinsäure in 100 g Toluol zu und rührt 2 h bei +10°C nach. Der ausgefallene Dicyclohexylharnstoff wird abfiltriert, das Filtrat zweimal mit je 150 ml 10 % Na₂ CO₃-Lösung und dreimal mit je 250 ml 30 % (NH₄)₂SO₄-Lösung gewaschen, 30 Min. mit 50 g wasserfreiem Na₂ SO₄ getrocknet und filtriert.

Bei 30°C Badtemperatur wird das Lösemittel in Vakuum abrotiert.

Man erhält 126 g einer viskosen Flüssigkeit mit einem Gehalt von 73 % Bis-(vinyladipoyl)-peroxid (VAP; 54 % der Theorie). Im Kühlschrank kristallisiert das Produkt.

### Beispiel 2

### 2.1 Herstellung von Adipinsäure-monovinylester-chlorid

In einem 2 l-Dreihalskolben mit Thermometer, Tropftrichter und Rückflußkühler werdenl045 g (= 6 Mol) Adipinsäure-monovinylester bei 50°C Badtemperatur aufgeschmolzen. 370 g (=2,7 Mol) Phosphortrichlorid werden bei 50°C langsam zugetropft und danach wird noch ca. 2 Std. bei 50°C reagieren gelassen, bis keine Gasentwicklung mehr auftritt.

Nach Abkühlen auf 25°C wird das rohe Säurechlorid von der phosphorigen Säure dekantiert. Am Rotationsdampfer wird im Wasserstrahl-Vakuum bei 50°C Badtemperatur das überschüssige PCl₃ abgezogen. Man erhält 1.127 g rohes Adipinsäure-monovinylester-chlorid.

### 2.2 Bis(vinyladipoyl)-peroxid aus dem Säurechlorid

In einer Lösung von 0,1 Mol (4 g) Ätznatron in 320 g Wasser löst man 0,6 Mol (64 g) Natriumcarbonat, danach 0,33 Mol (16 g) 70 % Wasserstoffperoxid. Unter Rühren kühlt man auf +5°C ab und tropft dann 0,5 Mol (96 g) Mono-vinyladipoylchlorid innerhalb 15 - 20 Minuten bei maximal 10°C zu.

Bei +5°C wird 1,5 h nachgerührt, der ausgefallene Feststoff wird dann abfiltriert und auf der Nutsche mit Wasser neutral und chloridfrei gewaschen.

Man erhält ca. 100 g eines wasserfeuchten Produkts mit einem Gehalt von ca. 80 % Bis-(vinyladipoyl)peroxid. (90 - 95 % Ausbeute).

### Beispiel 3

### Copolymerisation mit 1 % VAP

In einen Reaktor wurden 113 g Ethanol gegeben und mit N₂ gespült. 0,465 g Di-Myristylperoxydicarbonat (MYPC) wurden in etwas Vinylacetat gelöst und 69,3 g Vinylacetat und 0,7 g VAP zugegeben. Die Temperatur wurde auf 40°C eingestellt. Zur Überprüfung wurde jede Stunde eine Probe entnommen und in 15%iger Kochsalzlösung gefällt, gewaschen und getrocknet.

| Zeit | % Umsatz von Monomeren | AO-Gehalt % | %-Anteil an VAP |
|---|---|---|---|
| 3 h | 36 % | 0,089 % | 1,90 % |
| 4 h | 45 % | 0,077 % | 1,62 % |
| 5 h | 65 % | 0,068 % | 1,45 % |
| 6 h | 74 % | 0,65 % | 1,39 % |

### Beispiel 4

### Copolymerisation mit 3 % VAP

Die Durchführung ist dieselbe wie bei Beispiel 3.

| Zeit | % Umsatz von Monomeren | AO-Gehalt % | %-Anteil an VAP |
|---|---|---|---|
| 3 1/2 h | 44 % | 0,179 % | 3,7 % |
| 6 h | 67 % | 0,180 | 3,9 % |

### Beispiel 5

### Copolymerisation mit 5 % VAP

Die Durchführung ist dieselbe wie bei Beispiel 3.

| Zeit | % Umsatz von Monomeren | AO-Gehalt % | %-Anteil an VAP |
|---|---|---|---|
| 2 h | 25 % | 0,54 % | 11,5 % |
| 3 h | 43 % | 0,47 % | 10,2 % |
| 4 h | 58 % | 0,284 % | 6,1 % |

### Beispiel 6

### Ermittlung der Initiatoraktivität

Zur Ermittlung der Initiatoraktivität wurden 50 g Styrol mit 0,199 g LP bzw. 0,244 g VAP (70 %) versetzt und 4 h bei 90°C polymerisiert. Es wurde in ca. 80 g Toluol gelöst und in ca. 500 ml Ethanol gefällt und getrocknet bei 110°C.
- bei Lauroylperoxid (LP): Umsatz 38 %
- bei VAP: Umsatz 44 %

## Patentansprüche

1. Diacylperoxidvinylester der allgemeinen Formel I 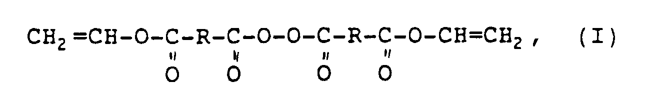 in der R einen Alkylenrest, der geradkettig, verzweigt oder cyclisch sein kann und 2 bis 10 C-Atome enthalten kann oder einen Arylrest bedeutet.

2. Verbindung nach Anspruch 1, mit R = n-Butylen.

3. Verbindung nach Anspruch 1, mit R = gegebenenfalls substituiertes Phenylen.

4. Verbindung nach Anspruch 1, mit R = Cyclohexylen.

5. Verfahren zur Herstellung einer Verbindung nach Anspruch 1 oder 2,
**dadurch gekennzeichnet,**
daß man eine Monovinyldicarbonsäure der Formel II 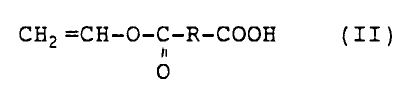 in der R die in einem der vorhergehenden Ansprüche angegebene Bedeutung hat mit mindestens äquimolaren Mengen an Dicyclohexylcarbodiimid und H₂O₂ und katalytischen Mengen eines aromatischen Amins unter Kühlung in einem apolaren aprotischen Lösungsmittel umsetzt.

6. Verfahren nach Anspruch 5,
**dadurch gekennzeichnet,**
daß man als Lösungsmittel Toluol verwendet.

7. Verfahren nach Anspruch 5 oder 6,
**dadurch gekennzeichnet,**
daß man als Katalysator Dimethylaminopyridin verwendet.

8. Verfahren nach einem der Ansprüche 5 bis 7,
**dadurch gekennzeichnet,**
daß man Monovinyladipinsäure, Monovinylterephthalsäure oder Monovinylcyclohexyl-1,4-dicarbonsäure verwendet.

9. Verfahren zur Herstellung einer Verbindung nach einem der Ansprüche 1 bis 4,
**dadurch gekennzeichnet,**
daß man Monovinyldicarbonsäurechlorid der Formel III 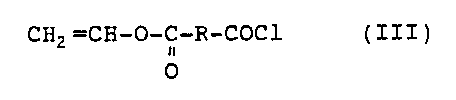 in der R die in einem der Ansprüche 1 bis 4 angegebene Bedeutung hat mit einer alkalischen wäßrigen H₂O₂-Lösung bei einer Temperatur von -10 bis +10°C umsetzt.

10. Verfahren nach Anspruch 9,
**dadurch gekennzeichnet,**
daß man das H₂O₂ in 20 bis 40 % Überschuß, bezogen auf das Säurechlorid, einsetzt.

11. Verfahren nach Anspruch 9 oder 10,
**dadurch gekennzeichnet,**
daß man Monovinyladipinsäurechlorid einsetzt.

12. Verwendung einer Verbindung nach einem der Ansprüche 1 bis 4 zur Copolymerisation mit olefinisch ungesättigten Monomeren.

13. Verwendung nach Anspruch 12 mit mindestens einem Monomeren aus der Gruppe Vinylacetat, Ethylen, Styrol, Vinylchlorid, Methacrylat, Methylmethacrylat und Vinylidenchlorid.

## Claims

1. Diacylperoxide vinyl esters of the general formula I 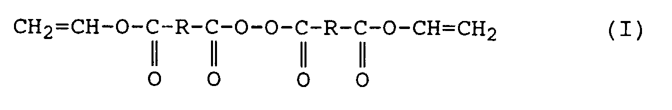 in which R denotes an alkylene residue which can be straight-chained, branched or cyclic and can contain 2 to 10 C atoms or it denotes an aryl residue.

2. Compound as claimed in claim 1, in which R = n-butylene.

3. Compound as claimed in claim 1, in which R = phenylene substituted if desired.

4. Compound as claimed in claim 1, in which R = cyclohexylen

5. Process for the production of a compound as claimed in claim 1 or 2,
**wherein**
a monovinyldicarboxylic acid of formula II 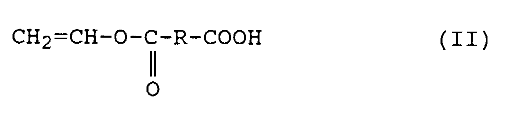 in which R has the meaning stated in one of the previous claims, is reacted with at least equimolar amounts of dicyclohexylcarbodiimide and H₂O₂ and catalytic amounts of an aromatic amine in an apolar aprotic solvent while cooling.

6. Process as claimed in claim 5,
**wherein**
toluene is used as the solvent.

7. Process as claimed in claim 5 or 6,
**wherein**
dimethylaminopyridine is used as the catalyst.

8. Process as claimed in one of the claims 5 to 7,
**wherein**
monovinyladipic acid, monovinylterephthalic acid or monovinylcyclohexyl-1,4-dicarboxylic acid is used.

9. Process for the production of a compound as claimed in one of the claims 1 to 4,
**wherein**
a monovinyldicarboxylic acid chloride of formula III 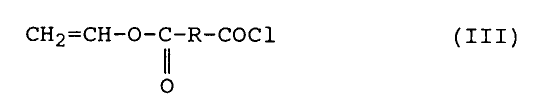 in which R has the meaning stated in one of the claims 1 to 4, is reacted with at an alkaline aqueous H₂O₂ solution at a temperature of -10 to +10°C.

10. Process as claimed in claim 9,
**wherein**
the H₂O₂ is used in a 20 to 40 % excess relative to the acid chloride.

11. Process as claimed in claim 9 or 10,
**wherein**
monovinyladipic acid chloride is used.

12. Use of a compound as claimed in one of the claims 1 to 4 for copolymerization with olefinic unsaturated monomers.

13. Use as claimed in claim 12 with at least one monomer from the group vinyl acetate, ethylene, styrene, vinyl chloride, methacrylate, methylmethacrylate and vinylidene chloride.

## Revendications

1. Vinylester de diacylperoxyde de la formule générale I 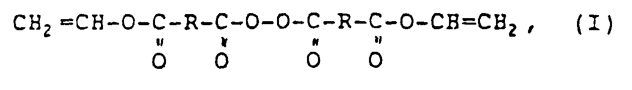 dans laquelle R signifie un radical alkylène, qui peut être linéaire, ramifié ou cyclique et peut contenir 2 à 10 atomes C ou un radical aryle.

2. Composé selon la revendication 1, avec R = n-butylène.

3. Composé selon la revendication 1, avec R = phénylène éventuellement substitué.

4. Composé selon la revendication 1, avec R = cyclohexylène.

5. Procédé pour la préparation d'un composé selon la revendication 1 ou 2,
caractérisé en ce qu'un acide monovinyldicarboxylique de la formule II 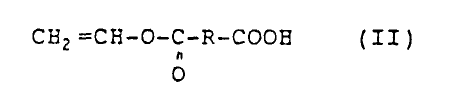 dans laquelle R a la signification indiquée dans l'une des revendications précédentes, est mis en réaction avec au moins des quantités équimolaires de dicyclohexylcarbodiimide et d'H₂O₂ et des quantités catalytiques d'une amine aromatique sous refroidissement dans un solvant aprotique apolaire.

6. Procédé selon la revendication 5,
caractérisé en ce qu'en tant que solvant, on utilise du toluène.

7. Procédé selon la revendication 5 ou 6,
caractérisé en ce qu'en tant que catalyseur, on utilise de la diméthylaminopyridine.

8. Procédé selon l'une des revendications 5 à 7,
caractérisé en ce que l'on utilise de l'acide monovinyladipinique, de l'acide monovinyltéréphtalique ou de l'acide monovinylcyclohexyl-1,4-dicarboxylique.

9. Procédé pour la préparation d'un composé selon l'une des revendications 1 à 4, caractérisé en ce que l'on met en réaction du chlorure de l'acide monovinyldicarboxylique de la formule III 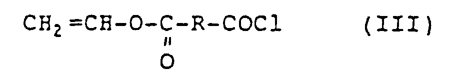 dans laquelle R a la signification indiquée dans les revendications 1 à 4 avec une solution alcaline aqueuse H₂O₂ à une température de -10 jusqu'à +10°C.

10. Procédé selon la revendication 9,
caractérisé en ce que l'on met en oeuvre l'H₂O₂ dans 20 à 40 % d'excédent rapportés au chlorure d'acide.

11. Procédé selon la revendication 9 ou 10,
caractérisé en ce que l'on met en oeuvre du chlorure de l'acide monovinyladipinique.

12. Utilisation d'une composition selon l'une des revendications 1 à 4 pour la copolymérisation avec des monomères oléfiniquement insaturés.

13. Utilisation selon la revendication 12 avec au moins un monomère du groupe acétate de vinyle, éthylène, styrène, chlorure de vinyle, méthacrylate, méthylméthacrylate et chlorure de vinylidène.
